# FASCICULE DE BREVET EUROPEEN

(11) **EP 3 759 153 B1**
(45) Date de publication et mention de la délivrance du brevet: **10.07.2024**
(21) Numéro de dépôt: 19707390.1
(22) Date de dépôt: 28.02.2019
(51) Int. Cl.: C08J 3/075, A61K 38/08, C07K 7/06, C07K 17/02

(54) **HYDROGEL POUR STIMULER LA NEUROTISATION, L'OSTEOGENESE ET L'ANGIOGENESE**
HYDROGEL ZUR FÖRDERUNG DER NERVENBILDUNG, DER KNOCHENBILDUNG UND DER GEFÄSSNEUBILDUNG
HYDROGEL FOR STIMULATING NEUROTIZATION, OSTEOGENESIS AND ANGIOGENESIS

(30) Priorité: 28.02.2018 FR 1851770
(43) Date de publication de la demande: 06.01.2021
(73) Titulaire: UNIVERSITE DE BORDEAUX, 33000 Bordeaux (FR); Institut Polytechnique de Bordeaux, 33400 Talence (FR); Institut National de la Santé et de la Recherche Médicale, 75013 Paris (FR); Centre National de la Recherche Scientifique, 75016 Paris (FR)
(72) Inventeur: AMEDEE, Joëlle, 33600 PESSAC (FR); LECOMMANDOUX, Sébastien, 33610 CANEJAN (FR); OLIVEIRA, Hugo, 33460 MACAU (FR); PAIVA DOS SANTOS, Bruno, 33000 BORDEAUX (FR); GARBAY, Bertrand, 33170 GRADIGNAN (FR); GARANGER, Elisabeth, 33400 TALENCE (FR)
(74) Mandataire: Cabinet Becker et Associés
(86) Numéro de dépôt international: PCT/EP2019/055075
(87) Numéro de publication internationale: WO 2019/166594

(56) Documents cités:
- WO-A1-2017/168183
- JP-A- 2017 093 315
- US-A1- 2011 200 560
- US-A1- 2016 193 384
- DIANA ISABEL SILVA ET AL: "Dorsal root ganglion neurons regulate the transcriptional and translational programs of osteoblast differentiation in a microfluidic platform", CELL DEATH & DISEASE, vol. 8, no. 12, 1 December 2017 (2017-12-01), XP055524607, DOI: 10.1038/s41419-017-0034-3
- ALEEZA FARRUKH ET AL: "Bifunctional Hydrogels Containing the Laminin Motif IKVAV Promote Neurogenesis", STEM CELL REPORTS, vol. 9, no. 5, 1 November 2017 (2017-11-01), United States, pages 1432 - 1440, XP055523676, ISSN: 2213-6711, DOI: 10.1016/j.stemcr.2017.09.002
- ISAAC L. MOSS ET AL: "A Novel Thiol-Modified Hyaluronan and Elastin-Like Polypetide Composite Material for Tissue Engineering of the Nucleus Pulposus of the Intervertebral Disc :", SPINE : AN INTERNATIONAL JOURNAL FOR THE STUDY OF THE SPINE, vol. 36, no. 13, 1 June 2011 (2011-06-01), US, pages 1022 - 1029, XP055524020, ISSN: 0362-2436, DOI: 10.1097/BRS.0b013e3181e7b705
- ROSINE PETITDEMANGE ET AL: "Tuning Thermoresponsive Properties of Cationic Elastin-like Polypeptides by Varying Counterions and Side-Chains", BIOCONJUGATE CHEMISTRY, vol. 28, no. 5, 18 April 2017 (2017-04-18), US, pages 1403 - 1412, XP055523687, ISSN: 1043-1802, DOI: 10.1021/acs.bioconjchem.7b00082
- YUAN ZOU ET AL: ""Click" chemistry in polymeric scaffolds: Bioactive materials for tissue engineering", JOURNAL OF CONTROLLED RELEASE, vol. 273, 1 March 2018 (2018-03-01), NL, pages 160 - 179, XP055524015, ISSN: 0168-3659, DOI: 10.1016/j.jconrel.2018.01.023

## Description

La présente invention concerne un hydrogel utile pour favoriser la neurotisation, l'ostéogenèse et l'angiogenèse.

### ARRIERE-PLAN TECHNOLOGIQUE

Dans le domaine de la médecine régénérative et de l'ingénierie tissulaire, le système nerveux périphérique reste, encore à ce jour, peu considéré dans un contexte de régénération du tissu osseux. Or, des données biologiques, expérimentales et cliniques démontrent des interactions entre les principaux événements de la reconstruction osseuse, à savoir sa néovascularisation, son innervation et la néo-formation osseuse.

Des données biologiques récentes obtenues par l'équipe des inventeurs (Silva et al, Cell Death and Disease, 2017 Dec 13;8(12):3209) ont démontré, à l'aide de modèles de co-cultures en deux dimensions de neurones sensoriels et de cellules mésenchymateuses, l'impact de la communication entre ces deux types cellulaires sur l'ostéogénèse. Il n'existe toutefois pas à l'heure actuelle de matériaux ou de matrices tridimensionnelles permettant de mettre ces observations en pratique, tant sur le plan expérimental que thérapeutique, en vue du développement d'un nouveau matériau innovant dédié à la régénération osseuse.

C'est dans ce contexte que les inventeurs ont développé un nouvel hydrogel, capable de recruter des neurones, notamment sensoriels, et d'héberger d'autres types cellulaires, et plus particulièrement des cellules ostéoformatrices et endothéliales.

### RESUME DE L'INVENTION

Un premier aspect de l'invention concerne un hydrogel comprenant :
i) un polypeptide de type élastine comprenant au moins un résidu alcénylé; et
ii) un peptide capable de recruter des cellules neuronales et/ou endothéliales, notamment un peptide IKVAV.

Selon un mode particulier de réalisation, l'hydrogel selon l'invention comprend :
i) un polypeptide de type élastine comprenant au moins un résidu alcénylé, notamment une méthionine alcénylé;
ii) un peptide capable de recruter des cellules neuronales et/ou endothéliales, notamment un peptide IKVAV ; et
iii) un polymère de réticulation, notamment un polymère de réticulation à terminaisons thiols.

Selon un mode particulier de réalisation, le polypeptide de type élastine est un polypeptide comprenant au moins une occurrence de la séquence VPGMG. De manière non-limitative, le peptide de type élastine peut notamment être le polypeptide MGTELAAASEFTHMW[VPGMG]₂₀ (ELP20), le polypeptide MW[VPGVGVPGMG(VPGVG)₂]₅ (ELPM20) ou le polypeptide MW[VPGVGVPGMG(VPGVG)₂]₁₀ (ELPM40).

Selon un mode particulier de réalisation, le peptide est un peptide IKVAV, notamment un peptide IKVAV de formule Cys-{Beta-Ala}-Ile-Lys-Val-Ala-Val-{Beta-Ala}-Cys.

Dans une autre variante, le polymère de réticulation, notamment un polymère de réticulation à terminaisons thiols, est un polymère multi-branché. Plus particulièrement, le polymère de réticulation peut être un poly(éthylène glycol) à 4 branches, notamment un poly(éthylène glycol) à 4 branches à terminaisons thiols, notamment un poly(éthylène glycol) PEG à 4 branches de masse moléculaire moyenne comprise entre 10 et 30 kDa, plus particulièrement un PEG à 4 branches à terminaisons thiols de masse moléculaire moyenne comprise entre 10 et 30 kDa. Le poly(éthylène glycol) à 4 branches , notamment le poly(éthylène glycol) à 4 branches à terminaisons thiols, peut notamment avoir une masse moléculaire moyenne de 20 kDa. Dans le contexte de la présente invention, la masse moléculaire moyenne est une masse moléculaire en masse.

Dans un mode particulier de réalisation, le polymère de réticulation à terminaisons thiols, le polypeptide type élastine comprenant un résidu méthionine alcénylé, et le peptide IKVAV sont présents dans l'hydrogel en un ratio thiol / alcène équimolaire.

Selon un autre mode particulier de réalisation, la concentration de l'hydrogel est comprise entre 5 et 15% en masse volumique (p/v), en particulier entre 7 et 8% (p/v).

Dans un autre mode de réalisation, le module de conservation G' de l'hydrogel est compris entre 1 et 5, de préférence entre 1 et 1,5 kPa.

L'hydrogel selon l'invention peut en outre comprendre au moins un agent actif sur le plan biologique, notamment au moins un facteur de croissance.

Un autre aspect de l'invention concerne l'hydrogel décrit dans la présente demande, pour son utilisation en tant que médicament.

Selon un autre aspect l'invention concerne l'hydrogel décrit dans la présente demande, pour son utilisation dans une méthode de régénération osseuse.

Par ailleurs, l'invention concerne également une méthode de culture cellulaire *in vitro,* comprenant la mise en culture de cellules dans un hydrogel tel que défini dans la présente demande.

### DESCRIPTION DES FIGURES

**Figure 1.** A)représentation schématique des composants de l'hydrogel et de la méthode de production ; B) photographie montrant un hydrogel ELPM40 + PEG. Barre = 1 mm.
**Figure 2****.** caractérisation rhéologique des hydrogels ELPM40 + PEG à différentes concentrations finales à 37 °C. Les modules élastiques (G') pour chaque concentration sont indiqués dans la figure.
**Figure 3****.** microscopie électronique à balayage de (A) ELPM40 + PEG, (B) ELPM40 + 25 % IKVAV, (C) ELPM40 + 25 % VKAIV, (D) ELPM40 + 50 % IKVAV, (E) ELPM40 + 50 % VKAIV montrant la structure poreuse de ces hydrogels. F) Quantification de la taille des pores : les compositions 25 % de peptide d'adhésion peptide ont des pores plus petits que les autres compositions.
**Figure 4****.** analyse *in vitro* de la dégradation des hydrogels par détermination des amines libres en solution après incubation avec de la protéinase K (0,5 U/mL) pendant 7 h.
**Figure 5****.** activité métabolique des cellules endothéliales (ECs), des cellules stromales mésenchymateuses de la moelle osseuse (BMSCs) et des neurones sensoriels (SNs). Toutes les compositions d'hydrogels ont permis l'attachement et la culture des trois types cellulaires.
**Figure 6****.** Morphologie des cellules endothéliales 7 jours après leur culture dans (A) ELPM40 + PEG, (B) ELPM40 + 25 % IKVAV, (C) ELPM40 + 25 % VKAIV, (D) ELPM40 + 50 % IKVAV et (E) ELPM40 + 50 % VKAIV. Dans toutes les compositions, les cellules ont pu entrer et migrer à l'intérieur des hydrogels et former différentes structures.
**Figure 7****.** cellules BMSC associées à (A) ELPM40 + PEG, (B) ELPM40 + 25 % IKVAV, (C) ELPM40 + 25 % VKAIV, (D) ELPM40 + 50 % IKVAV et (E) ELPM40 + 50 % VKAIV. Les cellules présentent une morphologie sphéroïde avec toutes les compositions d'hydrogels. (F) Détail de cellules plongées dans un hydrogel ELPM40 + 50 % IKVAV montrant deux noyaux connectés (indiqués par la flèche blanche), suggérant que les cellules peuvent proliférer à l'intérieur du gel.
**Figure 8****.** Morphologie et diffusion de neurones sensoriels cultivés dans (A) ELPM40 + PEG, (B) ELPM40 + 25 % IKVAV, (C) ELPM40 + 25 % VKAIV, (D) ELPM40 + 50 % IKVAV et (E) ELPM40 + 50 % VKAIV. Barres 100 µm. (F) Longueur moyenne des neurites mesurée pour toutes les compositions d'hydrogels.
**Figure 9****.** Expression de gène dans des cellules BMSC cultivées dans un milieu ostéogénique pendant 7 jours, en association avec différents hydrogels. L'expression est représentée par rapport à la composition ELPM40 + PEG, utilisée comme contrôle.
**Figure 10****.** Expression du gène *Tek* après 7 jours de culture de cellules endothéliales dans des compositions ELPM40 + PEG, ELPM40 + 25 % IKVAV, ELPM40 + 25 % VKAIV, ELPM40 + 50 % IKVAV ry ELPM40 + 50 % VKAIV.

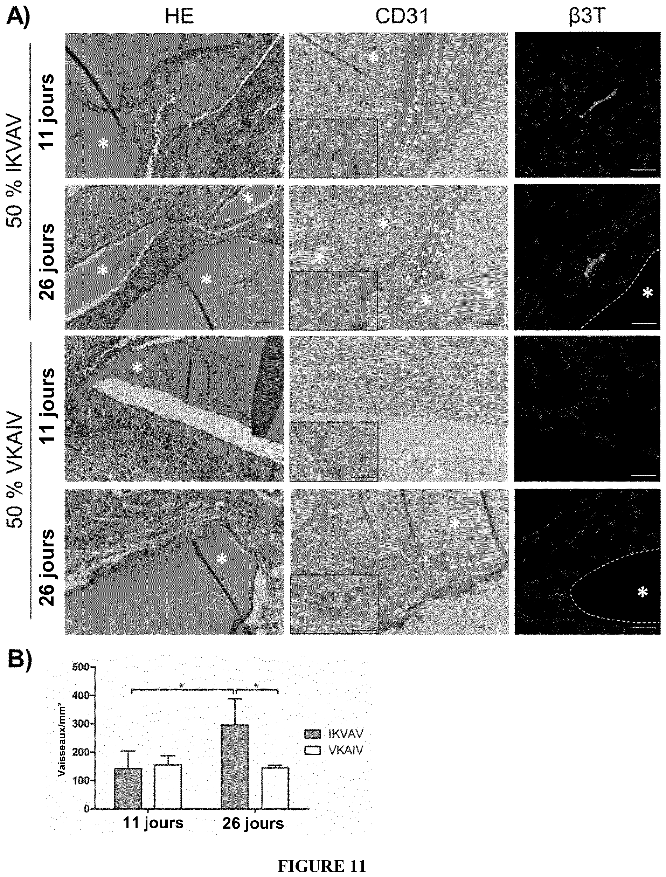

**Figure 11****.** Evaluation sous-cutanée d'une composition ELPM40 + 50 % IKVAV et ELPM40 + 50 % VKAIV après 11 et 26 jours. (A) des coupes histologiques ont été analysées pour déterminer i) le potentiel inflammatoire (HE), ii) la capacité à induire une angiogenèse (immunohistochimie de CD31) et iii) l'innervation (immunohistochimie de la tubuline βIII (β3T)). Barres = 50 µm pour HE et CD31. Des grossissements des colorations CD31 et β3T sont montrés, barres = 20 µm. (B) quantification des vaisseaux formés dans la région entourant la zone d'implantation d'une composition ELPM40 + 50 % et ELPM40 + 50 % VKAIV IKVAV.

### DESCRIPTION DETAILLEE DE L'INVENTION

La présente invention vise un hydrogel biocompatible capable de favoriser la neurotisation, l'ostéogenèse et l'angiogenèse. Cet hydrogel est caractérisé en ce qu'il comprend un polypeptide de type élastine comprenant au moins un résidu méthionine alcénylé; et un peptide capable de recruter des cellules neuronales et/ou endothéliales, notamment un peptide IKVAV. Plus particulièrement, l'hydrogel selon la présente invention est caractérisé en ce qu'il comprend i) un polypeptide de type élastine comprenant au moins un résidu méthionine alcénylé, et ii) un peptide capable de recruter des cellules neuronales et/ou endothéliales, notamment un peptide IKVAV et iii) un polymère de réticulation, notamment un polymère de réticulation à terminaisons thiols.

L'hydrogel selon l'invention peut notamment être formé au moyen d'un polymère de réticulation à terminaisons thiols. Dans le contexte de la présente invention, par « polymère de réticulation à terminaisons thiols » on entend un polymère présentant au moins une fonction thiol libre SH avant formation de l'hydrogel, c'est-à-dire avant la mise en contact avec le peptide de type élastine. Selon un mode particulier de réalisation, lorsque le ratio thiol/alcène est équimolaire ou lorsque le ratio thiol/alcène est supérieur à 1 (plus de thiols que d'alcène), le polymère à terminaison thiols dans l'hydrogel ne présente plus de fonction thiol SH libre après réaction avec l'ELP. Selon un autre mode de réalisation, lorsque le ratio thiol/alcène est inférieur à 1 (thiol en défaut ou alcène en excès), le polymère à terminaison thiols, dans l'hydrogel, peut présenter des fonctions thiol SH libres après réaction avec l'ELP. Ledit polymère est choisi parmi les polymères permettant la formation d'un hydrogel biocompatible, en ce sens qu'il n'est pas toxique pour les cellules. Il permet également avantageusement la diffusion d'oxygène et de nutriments, ainsi que celle de dioxyde de carbone et de déchets métaboliques pour alimenter les cellules et permettre leur survie. Les polymères de la solution d'hydrogel peuvent être d'origine naturelle, comme les protéines de matrice extracellulaire, ou synthétique comme le poly(éthylène glycol) (PEG), le poly(oxazoline) (POx) ou la poly(sarcosine) (PSar). Selon un mode de réalisation, le polymère de réticulation est plus particulièrement un polymère multi-branché, notamment un polymère linéaire ou un polymère multi-branché ayant au moins trois branches, plus particulièrement au moins quatre branches, ledit polymère linéaire multi-branché pouvant comprendre un groupement thiol à chacune de ses extrémités. Ainsi, le polymère de réticulation peut plus particulièrement être un polymère multi-branché, notamment un polymère linéaire ou un polymère multi-branché ayant au moins trois branches, plus particulièrement au moins quatre branches, ledit polymère linéaire multi-branché comprenant un groupement thiol à chacune de ses extrémités. Selon un mode particulier de réalisation, le polymère multi-branché est un polymère à quatre branches, pouvant notamment comprendre un groupement thiol à l'extrémité de chacun de ses branches. Peuvent notamment être cités les polymères de type poly(éthylène glycol) (ou PEG) linéaires ou à trois ou quatre branches, ou plus de quatre branches, plus particulièrement quatre branches. Selon un mode de réalisation, sont mis en oeuvre des polymères de type poly(éthylène glycol) (ou PEG) linéaires ou à trois ou quatre branches, ou plus de quatre branches, plus particulièrement quatre branches, chaque extrémité des PEG linéaires comprenant un groupement thiol, ou chaque branches des PEG multi-branché comprenant à leurs extrémités un groupement thiol. Dans un mode de réalisation, l'hydrogel comprend un PEG à quatre branches comprenant chacun à son extrémité un groupement thiol, la masse moléculaire moyenne dudit PEG étant comprise entre 1 et 100 kDa, plus particulièrement entre 10 et 30 kDa, le PEG ayant de manière encore plus particulière une masse moléculaire moyenne de 20 kDa.

Le second composant de l'hydrogel selon l'invention est un polypeptide de type élastine (ou ELP pour Elastin-Like Polypeptide en anglais) comprenant au moins un résidu méthionine alcénylé. Ce type de polypeptides, leur procédé de fabrication par génie génétique, et leur purification sont connus de l'homme du métier qui peut notamment se référer à la demande internationale WO2017021334 et aux articles Petitdemange et al. (Biomacromolecules. 2017 Feb 13;18(2):544-550) et Petitdemange et al. (Bioconjug Chem. 2017 May 17;28(5):1403-1412). Dans le cadre de la présente invention, le terme "résidu méthionine alcénylé" signifie que la chaîne latérale du résidu méthionine est liée de manière covalente à un groupement comprenant un groupe alcène, c'est-à-dire comprenant au moins une double liaison entre deux atomes de carbone. De manière préférée, le terme "groupe alcène" se réfère à la présence d'un groupe -CH=CH2 dans le groupement lié au résidu méthionine. Selon un mode particulier de réalisation, le groupement méthionine est lié au groupement de formule (I):

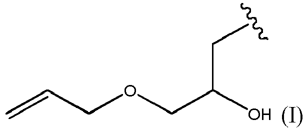

Selon un mode de réalisation, la synthèse d'un ELP alcénylé au moyen du groupement de formule (I) peut être réalisée par thioalkylation chimiosélective au niveau de chaînes latérales méthionines en utilisant un éther d'allyle et glycidyle selon la procédure décrite dans Petitdemange et al. (Bioconjug Chem. 2017 May 17;28(5):1403-1412).

Selon un mode de réalisation, l'ELP alcénylé mis en oeuvre dans le cadre de la présente invention comprend au moins une occurrence de la séquence d'acides aminés VPGMG dans laquelle le résidu méthionine est alcénylé.

Dans un mode de réalisation, l'ELP alcénylé a une structure de formule (II)

Z-[VPGXG]ₙ-OH (II)

dans laquelle:
Z est un peptide comprenant entre 1 et 20 acides aminés;
X représente un résidu glycine, un résidu valine ou un résidu méthionine alcénylé, notamment un résidu méthionine alcénylé de formule (III): 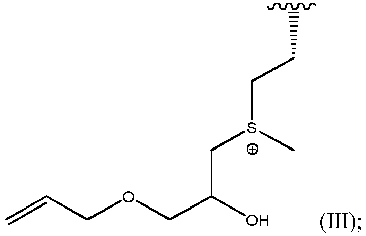
n est un nombre entier compris entre 1 et 200, plus particulièrement entre 10 et 200, de manière encore plus particulière entre 15 et 50, notamment entre 20 et 40; et
dans laquelle le rapport entre le ratio molaire valine / méthionine alcénylée en position X est compris entre 0:1 et 10:1, plus particulièrement entre 1:1 et 5:1, ledit ratio étant plus particulièrement de 3:1.

Selon un mode de réalisation, X représente un résidu glycine ou un résidu méthionine alcénylé, notamment un résidu méthionine alcénylé de formule (III).

Selon un autre mode de réalisation, préféré, X représente un résidu valine ou un résidu méthionine alcénylé, notamment un résidu méthionine alcénylé de formule (III).

Selon un mode de réalisation, n est un nombre entier compris entre 30 et 50, en particulier entre 35 et 45, n étant plus particulièrement égal à 35, 36, 37, 38, 39, 40, 41, 42, 43, 44 ou 45. Plus particulièrement, n est égal à 40.

Selon un mode particulier de réalisation, Z est un peptide dont le résidu d'acide aminé à l'extrémité amino-terminale est une méthionine.

Selon un mode particulier de réalisation, Z ne comprend pas la séquence d'acides aminés IKVAV. Selon un autre mode de réalisation, les acides aminés compris dans Z immédiatement en amont du motif [VPGXG]ₙ correspondent au dipeptide MW. Z peut notamment être constitué du dipeptide MW ou comprendre ce dipeptide. A titre illustratif, le peptide ELP20 décrit ci-dessous comprend une séquence Z dans laquelle le dipeptide MW se trouve à son extrémité C-terminale dans la séquence MGTELAAASEFTHMW.

Selon un mode de réalisation, l'ELP employé est un peptide de formule Z-[VPGXG]ₙ dans laquelle X est une méthionine alcénylée.

Selon un autre mode de réalisation l'ELP employé est un peptide de formule Z-[VPGXG]ₙ dans laquelle le rapport entre le ratio molaire valine / méthionine alcénylée en position X est compris entre 0:1 et 10:1, plus particulièrement entre 1:1 et 5:1, ledit ratio étant plus particulièrement de 3:1.

Selon un autre mode de réalisation l'ELP employé est un peptide de formule Z-[VPGVGVPGMG(VPGVG)₂]ₓ, en particulier MW[VPGVGVPGMG(VPGVG)₂]ₓ dans laquelle x est un nombre entier compris entre 2 et 15, plus particulièrement entre 5 et 10.

Selon un mode de réalisation, l'ELP employé est dérivé d'un peptide choisi parmi le peptide MGTELAAASEFTHMW[VPGMG]₂₀ (ELP20), le peptide MW[VPGVGVPGMG(VPGVG)₂]5 (ELPM20) ou le peptide MW[VPGVGVPGMG(VPGVG)₂]₁₀ (ELPM40) décrits dans WO2017021334, ledit peptide comprenant au moins un résidu méthionine alcénylé.

Selon un mode particulier de réalisation, l'ELP a la structure suivante: 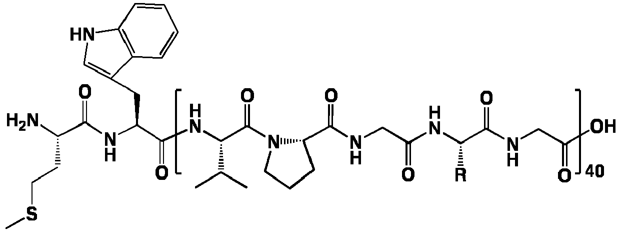 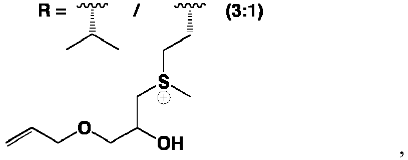 plus particulièrement la structure MW[VPGVGVPGMₐG(VPGVG)₂]₁₀ (ELP-M(alcène)-40), où Mₐ représente le résidu méthionine alcénylé de formule (III) ci-dessus.

Selon une variante de réalisation de tous les ELP décrit ci-dessus, la méthionine amino-terminale desdits ELP est une méthionine alcénylée, notamment une méthionine alcénylée de formule (III) ci-dessus. De manière illustrative, l'ELP peut notamment avoir la structure suivante:

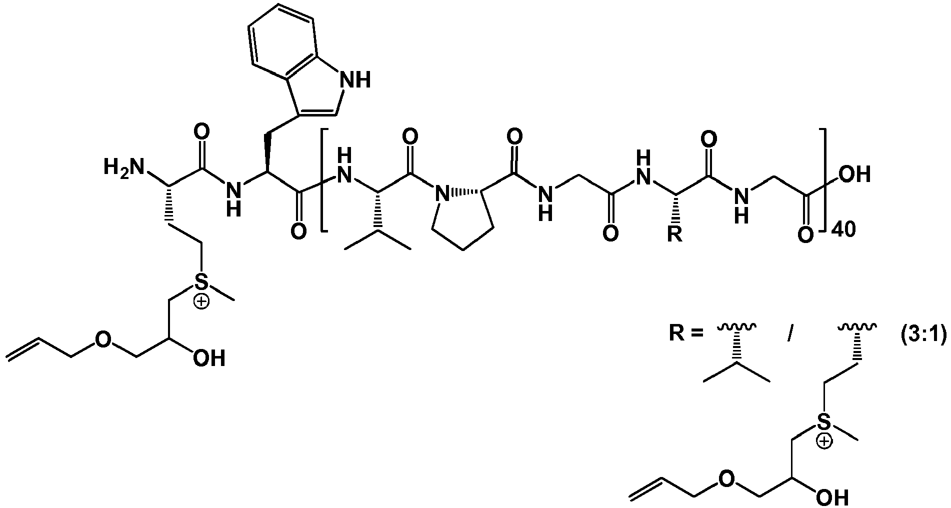

Dans un mode de réalisation, cette structure peut être représentée alternativement selon la formule suivante:

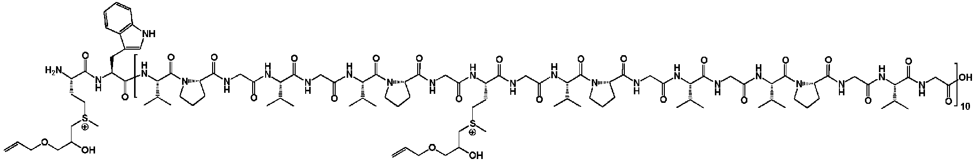

Le composant iii) de l'hydrogel est un peptide capable de recruter des cellules neuronales et/ou endothéliales. On peut notamment citer à titre de peptides capables de recruter des cellules endothéliales les peptides REDV, RGD et GRGDSP dérivés de la fibronectine, IKLLI, IKVAV, PDSGR et YIGSR, dérivés de la laminine, et le peptide DGEA dérivé du collagène de type I. A titre de peptides capables de recruter des cellules neuronales, nous pouvons citer, notamment, les peptides YIGSR, RNIAEIIKDI et IKVAV dérivés de la laminine. Selon un mode particulier de réalisation, le peptide capable de recruter des cellules neuronales et/ou endothéliales et un peptide IKVAV, c'est-à-dire un peptide comprenant la séquence d'acides aminés IKVAV dérivée de la laminine A. Dans un mode particulier de réalisation, le peptide capable de recruter des cellules neuronales et/ou endothéliales inclu dans l'hydrogel de l'invention est un peptide, notamment un peptide comprenant la séquence IKVAV, comprenant des résidus cystéine à chacune de ses extrémités. Les résidus cystéines peuvent être liés de manière covalente directement à la séquence d'acides aminés IKVAV, ou par l'intermédiaire d'espaceurs. Selon un mode particulier de réalisation, les résidus cystéine sont liés au peptide capable de recruter des cellules neuronales et/ou endothéliales, notamment à un peptide IKVAV, par l'intermédiaire d'un espaceur, notamment un espaceur peptidique ou pseudopeptidique. L'espaceur peut notamment être un acide aminé ou une séquence d'acides aminés (notamment un diou tripeptide), notamment un acide aminé bêta, plus particulièrement un acide aminé bêta-Ala. Ainsi, selon un mode particulier de réalisation, le peptide capable de recruter des cellules neuronales et/ou endothéliales est un peptide IKVAV de formule Cys-{espaceur}-Ile-Lys-Val-Ala-Val-{espaceur}-Cys, notamment le peptide de formule Cys-{Beta-Ala}-Ile-Lys-Val-Ala-Val-{Beta-Ala}-Cys.

La quantité des composants de l'hydrogel peut varier dans une large mesure, pour autant que l'hydrogel résultant favorise la neurotisation, l'ostéogenèse et/ou l'angiogenèse. Selon un mode particulier de réalisation, les différents composants sont en une quantité respectant un ratio molaire thiol / alcène compris entre 10:1 et 1:10, notamment entre 5:1 et 1:5, en particulier entre 2:1 et 1:2. Selon un mode particulier de réalisation, le ratio thiol / alcène est équimolaire (i.e. il correspond à un ratio molaire 1:1). Il est entendu que les groupements thiols sont présents sur le polymère de réticulation et/ou sur le peptide capable de recruter des cellules neuronales et/ou endothéliales, notamment un peptide IKVAV tel que décrit ci-dessus. Ainsi, l'hydrogel peut comprendre des quantités variables de chacun de ses composants, tout en respectant les ratios définis ci-avant. Par exemple, les groupes thiols portés par le peptide IKVAV peuvent représenter entre 10 et 75 % en moles de l'ensemble des groupes thiols apportés par le peptide IKVAV et le polymère de réticulation, plus particulièrement entre 20 et 60 % en moles, en particulier entre 25 et 50 % en moles de l'ensemble des groupes thiols apportés par le peptide IKVAV et le polymère de réticulation. Selon un mode particulier de réalisation, les groupes thiols portés par le peptide IKVAV représentent 25 % en moles de l'ensemble des groupes thiols apportés par le peptide IKVAV et le polymère de réticulation. Selon un autre mode particulier de réalisation, les groupes thiols portés par le peptide IKVAV représentent 50 % en moles de l'ensemble des groupes thiols apportés par le peptide IKVAV et le polymère de réticulation.

Les proportions des composants de l'hydrogel sont également choisies de manière à obtenir un hydrogel présentant des propriétés rhéologiques adaptées au développement de cellules, notamment de cellules neuronales, osseuses ou endothéliales, en particulier de cellules neuronales. L'invention permet ainsi d'adapter de manière très fine la structure de l'hydrogel au(x) type(s) cellulaire(s) dont le développement doit être favorisé. Selon un mode de réalisation, la rigidité de l'hydrogel répond au paramètre de module de conservation G' compris dans l'intervalle suivant: 1 kPa < G' < 5 kPa, en particulier 1 kPa < G' < 1,5 kPa.

Selon un autre mode particulier de réalisation, la concentration de l'hydrogel est comprise entre environ 5 et environ 15 % en masse volumique (p/v), en particulier entre environ 7 et environ 8 % (p/v), cette masse volumique étant plus particulièrement égale à environ 7,5 % (p/v).

Par ailleurs, l'hydrogel selon l'invention présente une microporosité, et peut comprendre des pores de taille moyenne variant notamment entre 5 et 20 µm, plus particulièrement entre 10 et 17 µm.

Selon un mode particulier de réalisation, l'hydrogel de l'invention peut comprendre un ou plusieurs autres éléments, d'autres séquences peptidiques pour cibler d'autres fonctions, ou encore des agents bioactifs tels que des facteurs de croissance, notamment pour stimuler d'autant plus la neurotisation, l'ostéogenèse et/ou l'angiogenèse. Cependant, selon un mode particulier de réalisation, l'hydrogel est dépourvu de facteurs de croissance ou n'en contient qu'à hauteur de 0 à 10 % en poids par rapport au poids total de l'hydrogel, plus particulièrement de 0 à 5%, de manière encore plus particulière de 0 à 1%, de manière particulière de 0 à 0,1 % en poids par rapport au poids de l'hydrogel. Comme nous le verrons ci-dessous, l'hydrogel peut également être utilisé comme véhicule de thérapie cellulaire. Ainsi, un hydrogel tel que défini ci-dessus peut également comprendre des cellules d'intérêt thérapeutique, par exemple des cellules souches, notamment des cellules souches induites vers un lignage d'intérêt, des cellules souches hématopoïétiques, des cellules souches mésenchymateuses stromales dérivées de la moelle osseuse ou du tissu adipeux, des cellules souches neuronales, ou un mélange de cellules de lignages différents pour stimuler un processus de communication cellulaire. Dans un mode particulier de réalisation, les cellules sont des cellules souches, à l'exclusion des cellules souches embryonnaires humaines. Les cellules sont introduites dans l'hydrogel après formation dudit hydrogel, par mise en contact des cellules et de l'hydrogel et mise en culture pendant un temps suffisant (notamment pendant au moins 1, 2, 3, 4, 5, 6 ou au moins 7 jours) pour que les cellules puissent coloniser l'hydrogel. L'hydrogel selon l'invention peut également inclure des nanoparticules de composants minéraux, notamment de l'hydroxyapatite ou du phosphate de calcium (notamment en une quantité comprise entre 10 et 40 % (p/v)), afin d'augmenter le potentiel ostéogénique de l'hydrogel.

L'hydrogel de l'invention peut être fabriqué par mélange de ces différentes composants i) à iii), et de tout autre élément optionnel tel que des facteurs de croissance. Les composants i) à iii) et la quantité de ces composants i) à iii) sont choisis afin de préparer un hydrogel présentant des propriétés physiques et de support adaptées à la problématique posée à son utilisateur. Avantageusement, la formation de l'hydrogel par réticulation est réalisée sous l'action d'un stimulus tel qu'une modification de température, de pH, ou au moyen d'un agent de réticulation, notamment un agent de réticulation photosensible (ou photoinitiateur). On peut ainsi citer à titre illustratif l'induction d'une photopolymérisation au moyen d'un photoinitiateur tel que le composé Irgacure 2959, notamment utilisé à une masse volumique de 0,5 % (p/v) dans le mélange, et activé par lumière UV-visible (λ=305 - 405 nm, notamment à 305 nm). Dans une autre variante, le photoinitiateur peut être notamment choisi parmi le phényl-2,4,6-triméthylbenzoylphosphinate de lithium (LAP) et la riboflavine. La concentration de LAP peut varier de 0,005 % à 0,5 % (p/v) dans le mélange, et sa photoinitiation peut être déclenchée à une longueur d'onde comprise entre 365 et 475 nm.

Selon un aspect, l'invention concerne un hydrogel susceptible d'être obtenu, ou obtenu, par un procédé comprenant les étapes suivantes :
(a) le mélange de
   i) un polypeptide de type élastine comprenant au moins un résidu méthionine alcénylé et
   ii) un peptide capable de recruter des cellules neuronales et/ou endothéliales, notamment un peptide IKVAV, et
      éventuellement (iii) un polymère de réticulation à terminaisons thiols ; et
   iv) un initiateur de polymérisation, notamment un photoinitiateur (et éventuellement d'un autre composant tel qu'un ou plusieurs facteurs de croissance); et
b) l'application d'un stimulus, notamment d'un rayonnement lumineux, notamment UV, pour activer la polymérisation.

Avantageusement, comme nous l'indiquions ci-dessus, les propriétés rhéologiques de l'hydrogel peuvent être très finement définies. Par ailleurs, les inventeurs ont pu montrer que l'hydrogel selon l'invention est dégradable et présente une structure poreuse. Enfin, les inventeurs ont pu démontrer que cet hydrogel est adapté à la culture de types cellulaires très différents, à savoir des neurones, des cellules mésenchymateuses, des cellules osseuses, des cellules endothéliales ou leurs progéniteurs, que ces cellules peuvent migrer à l'intérieur de la structure de l'hydrogel, et qu'il n'a pas d'effet cytotoxique. Il rassemble ainsi toutes les propriétés avantageuses utiles au développement d'un outil adapté à la régénération tissulaire.

L'hydrogel selon l'invention est donc capable de supporter efficacement la culture *in vitro* de différents types cellulaires. En conséquence, selon un mode particulier de réalisation, l'invention concerne un nouveau support tridimensionnel capable d'héberger *in vitro* différentes cellules d'intérêt pour la régénération osseuse, notamment neuronales, osseuses ou endothéliales. L'invention met donc à disposition de l'homme du métier un système particulièrement avantageux de culture cellulaires 3D permettant aux cellules de se développer dans un environnement favorable, mais également d'étudier les interactions de différents types cellulaires entre eux. Ce paramètre est important pour étudier les phénomènes de régénération qui peuvent nécessiter des dialogues complexes entre différents types cellulaires. Le support de l'invention peut notamment être utilisé pour héberger des cellules ostéoformatrices et endothéliales, et pour étudier l'effet angiogénique, ostéogénique et de neurotisation dans une méthode de culture cellulaire *in vitro,* comprenant la mise en culture de cellules dans un support tel que défini ci-dessus. L'utilisation du support selon l'invention peut comprendre par ailleurs l'ajout d'un agent à la culture, tel qu'un facteur de croissance ou toute autre agent ayant un effet biologique ou susceptible d'avoir un effet biologique (agent candidat) pour déterminer son effet sur un ou plusieurs paramètres et réponses cellulaires tels que la croissance des cellules, l'induction d'une quiescence, d'une mort cellulaire, de la sécrétion de protéine, ou d'autres molécules, ou d'ions (notamment ions calcium, potassium), ou encore l'expression de certains gènes.

Selon un autre aspect, l'hydrogel de l'invention est utilisé dans une méthode de traitement, notamment comme implant. L'hydrogel est notamment utilisé dans une méthode de traitement de médecine regénérative. Il peut notamment être utilisé pour stimuler l'innervation d'un tissu, notamment un tissu osseux, et est notamment utilisable en ingénierie osseuse. Avantageusement, l'hydrogel selon l'invention favorise la neurotisation, notamment dans un contexte de régénération. Plus particulièrement, l'hydrogel selon l'invention peut avantageusement être utilisé pour recruter et stimuler le système nerveux sensoriel, plus particulièrement pour favoriser la régénération osseuse. L'hydrogel selon l'invention peut également être employé afin d'optimiser ou de restaurer la vascularisation et l'innervation d'un tissu.

Selon un autre mode de réalisation, l'hydrogel peut être utilisé comme véhicule de thérapie cellulaire. Ainsi, un hydrogel tel que défini ci-dessus préalablement colonisé par des cellules d'intérêt thérapeutique, par exemple au moyen de cellules souches, notamment de cellules souches induites dans un lignage d'intérêt, des cellules souches hématopoïétiques, des cellules souches mésenchymateuses stromales dérivées de la moelle osseuse ou du tissu adipeux, des cellules souches neuronales, ou un mélange de cellules de lignages différents. Un tel hydrogel est utilisable dans une méthode de traitement par régénération cellulaire ou tissulaire.

L'hydrogel selon l'invention peut également être utilisé pour modifier des systèmes d'implant, pour améliorer leur biocompatibilité et leur intégration.

### EXEMPLES

### Production du peptide ELP-M(alcène)-40 :

L'article Petitdemange et al. (Biomacromolecules. 2017 Feb 13;18(2):544-550. doi:10.1021/acs.biomac.6b01696. Epub 2017 Jan 27. PubMed PMID: 28075561) décrit la construction du vecteur d'expression du peptide MX[VPGVGVPGMG(VPGVG)₂]₁₀ (ELPM40), son expression dans *E. coli,* son isolement à partir des lysats bactériens, sa purification et sa caractérisation.

La méthode de production du peptide ELP-M(alcène)-40, de structure : 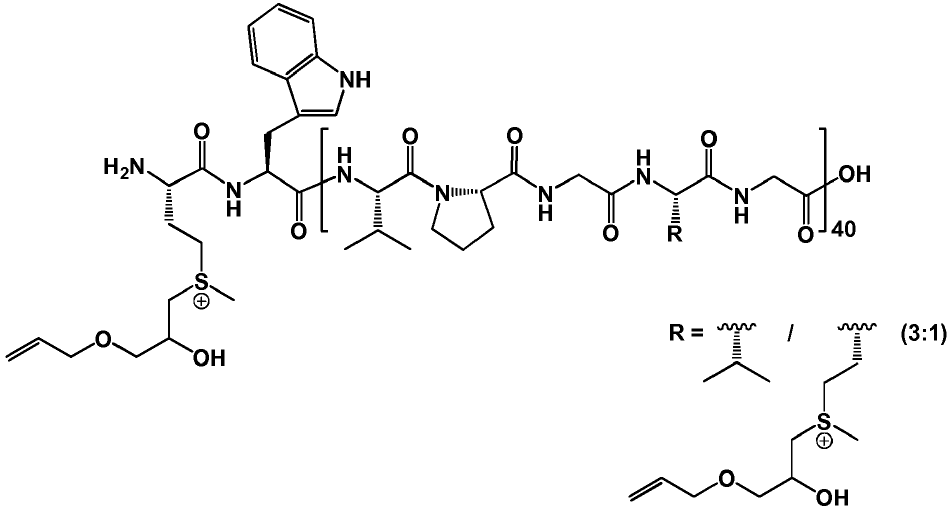 par thioalkylation chimiosélective des chaînes latérales méthionine du peptide ELPM40 en utilisant l'éther d'allyle et glycidyle est décrite dans Petitdemange et al. (Bioconjug Chem. 2017 May 17;28(5):1403-1412. doi: 10.1021/acs.bioconjchem.7b00082. Epub 2017 Apr 18. PubMed PMID: 28381088).

Dans la suite de la partie expérimentale, le terme ELPM40 est utilisé pour désigner le peptide ELP-M(alcène)-40.

### Développement d'un hydrogel composite

### Production de l'hydrogel

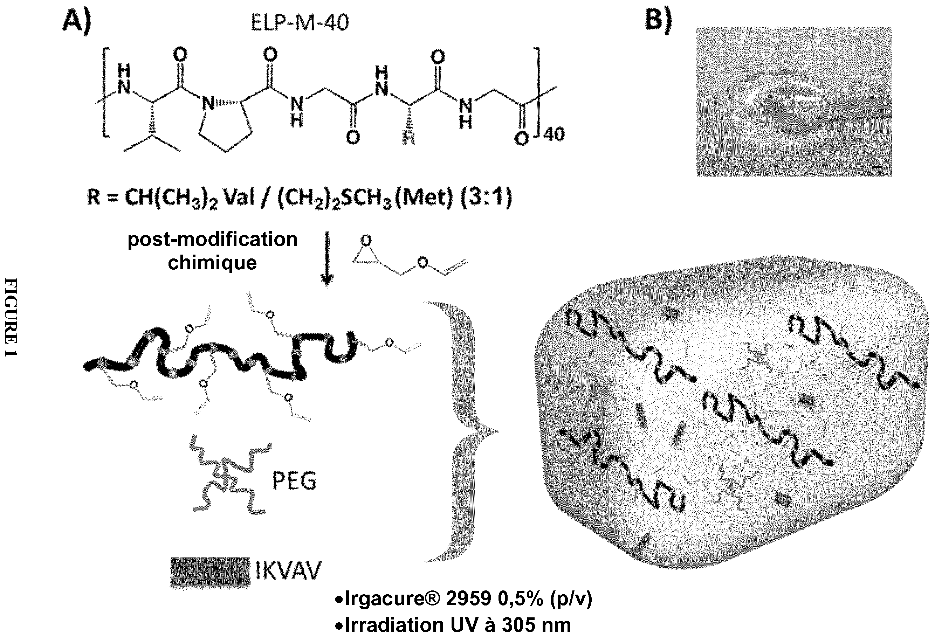

Les hydrogels produits contiennent le peptide ELPM40, un PEG à terminaisons thiols (SH-PEG, 20 kDa ; JenKem, USA) et le peptide d'adhésion Cys-{Beta-Ala}-Ile-Lys-Val-Ala-Val-{Beta-Ala}-Cys (IKVAV) ou une version aléatoire Cys-{Beta-Ala}-Val-Lys-Ala-Ile-Val-{Beta-Ala}-Cys (VKAIV), à un ratio thio / alcène équimolaire. La photopolymérisation a été réalisée en utilisant le photoinitiateur Irgacure 2959 (0,5 % p/v) exposé à une lumière UV de longueur d'onde 305 nm pendant 8 minutes, à la suite d'une réaction thiol-ène. La figure 1 montre une représentation schématique de l'hydrogel ainsi obtenu.

Différents ratio en masse d'ELP et de PEG ont été testés pour obtenir une rigidité optimale et un attachement de neurones sensoriels et une excroissance de neurites. Brièvement, le SH-PEG a été substitué par le peptide d'adhésion à différentes proportions. Les compositions testées sont les suivantes :
(i) ELPM40 + PEG, où 100 % (mol) des groupes thiols sont représentés par le SH-PEG,
(ii) ELPM40 + 25 % IKVAV ou VKAIV, où 25 % (mol) des groupes thiols sont représentés par le peptide d'adhésion, et
(iii) ELPM40 + 50 % IKVAV or VKAIV, où 50 % (mol) des groupes thiols sont représentés par le peptide d'adhésion.

### Propriétés rhéologiques

Les propriétés rhéologiques des hydrogels ont été évaluées après 24h de trempage dans du PBS, par mesure de la dépendance de fréquence des modules élastique (G') et de perte (G"). Des balayages de fréquences ont été réalisés sur des compositions ELPM40 + PEG à différentes concentrations d'hydrogel, i.e. à 5 %, 7,5 %, 10 % and 15 % (p/v). Les mesures ont été réalisées à 37°C.

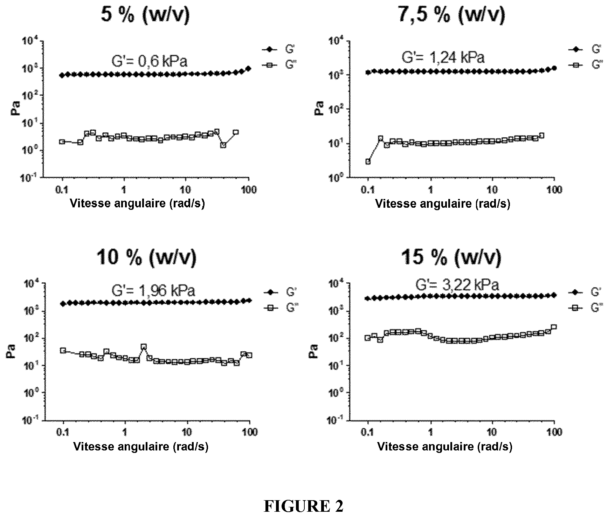

Nous avons pu montrer que le module élastique augmentait de manière proportionnelle à la concentration finale de l'hydrogel (figure 2).

### Analyse structurale par microscopie électronique à balayage

Une analyse par cryomicroscopie électronique à balayage a été réalisée pour déterminer la taille des pores des hydrogels produits à différentes concentrations, et visualiser leur structure.

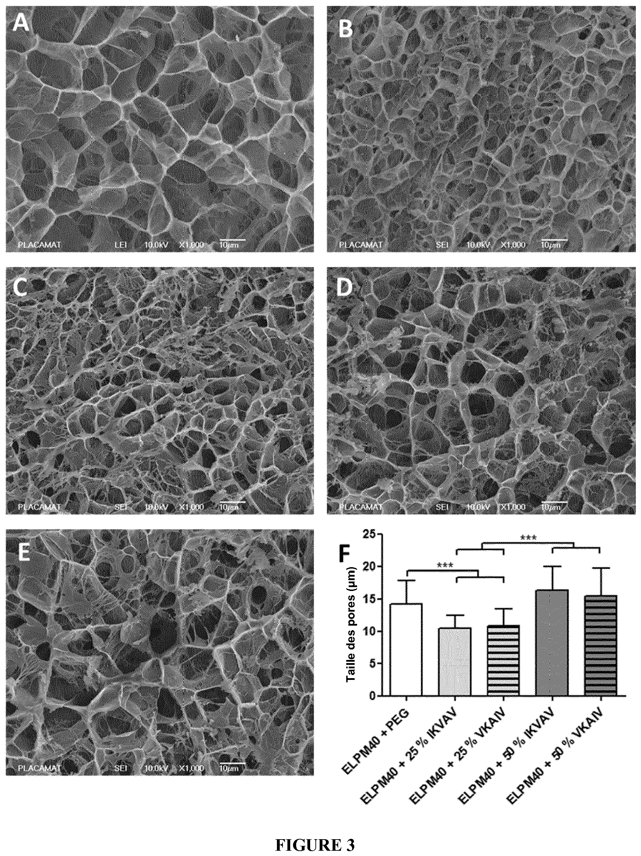

Toutes les compositions d'hydrogels présentent une structure poreuse (figures 3A-E), avec une taille de pores variant de 10,49 ± 1,61 µm (ELPM40 + 25 % IKVAV) à 16,39 ± 2,81 µm (ELPM40 + 50 % IKVAV). Les compositions d'hydrogels comprenant 25 % de peptide d'adhésion présentent des tailles de pores plus petites en comparaison des compositions obtenues dans les autres conditions (figure 3F).

### Analyse de dégradation in vitro de l'hydrogel :

15 µL d'hydrogel ont été incubés dans 150 µL d'une solution de protéinase K (0,5 U/mL) (Amresco, #0706) dans un tampon 50 mM Tris Base, 1 mM EDTA, 5 mM CaCl₂ et 0,5 % (v/v) Triton X-100 (pH 8,0) pendant 7 h à 37 °C. Le contenu en groupes amines libres, exprimé par le nombre de groupes amines libres présents pour 1000 acides aminés (n/1000) a été déterminé en utilisant de l'acide 2,4,6-trinitrobenzenesulfonique (TNBS) selon la procédure décrite dans Gilbert et al. J Biomed Mater Res 1990, 24, 1221. Le contenu en groupes amines libres a été calculé en utilisant un coefficient d'absorption molaire de 14600 L/mol/cm pour la trinitrophényl lysine (Wang et al., Biochim Biophys Acta 1978, 544, 555).

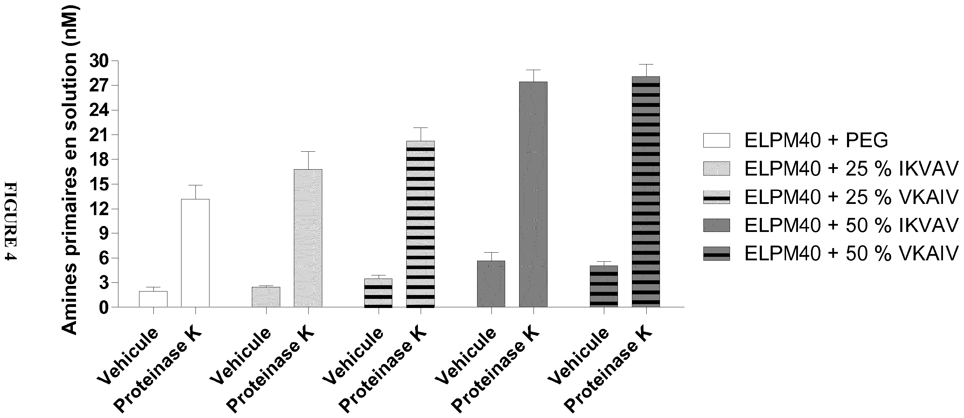

Après incubation avec la protéinase K, des amines primaires libres en solution ont pu être détectées pour toutes les compositions d'hydrogels (figure 4), indiquant que le processus de réticulation n'a pas interféré avec les propriétés de dégradation *in vitro.* Il s'agit d'une propriété importante pour des matériaux ayant des applications biomédicales. Différents types cellulaires doivent pouvoir entrer dans la structure de l'hydrogel, et y migrer. Le mécanisme le plus commun de migration cellulaire est la sécrétion de protéases qui digèrent la structure d'hydrogel à faible vitesse, ce qui permet la colonisation de l'hydrogel par les cellules.

### Evaluation biologique au moyen de cellules primaires

### Isolement des cellules et culture :

Des neurones sensoriels (SN) primaires ont été obtenus à partir de ganglions de la racine dorsale (DRG) de rats Wistar âgés de 6 à 10 semaines, selon une procédure décrites par Malin et al., Nat Protoc 2007, 2, 152.

Des cellules stromales mésenchymateuses dérivées de la moelle osseuse (BMSC) ont été isolées de rats Wistar âgés de 6 à 10 semaines. En bref, les fémurs et les tibias des animaux ont été prélevés et découpés à leurs extrémités pour exposer la moelle osseuse. Les os ont été transférés dans des tubes de 1,5 mL puis centrifugés à 1500 x g pendant 30 s pour en expulser la moelle osseuse. Les culots obtenus ont ensuite été resuspendus dans du DMEM à faible teneur en glucose (Gibco) supplémenté de 10 % (v/v) de sérum de veau foetal (PANTM-Biotech, Aidenbach, Allemagne) et de 1 % (v/v) de pénicilline / streptomycine, puis passés au travers d'aiguilles 16G et 21G 4 à 6 fois. Le contenu obtenu à partir d'un fémur et d'un tibia a ensuite été ensemencé dans une flasque de 75 cm² et incubé dans un incubateur humidifié (37°C et 5 % CO₂). Le milieu de culture a été changé deux fois pas semaine pour retirer les cellules non-adhérentes. Les cellules adhérentes furent cultivées jusqu'à atteindre 90 % de confluence, puis transférées dans des récipients de surface plus importante. Les cellules ont été utilisées jusqu'au passage P3. Lorsqu'elles ont été associées aux hydrogels, les BMSC ont été cultivées dans un milieu ostéogénique, qui correspond au milieu de culture mentionné ci-dessus, supplémenté de 10⁻⁹ M de déxaméthasone (Sigma-Aldrich) 10 mM de β-glycerophosphate (Sigma-Aldrich) et 50 µg/mL d'acide ascorbique (Sigma-Aldrich)

Les cellules souches endothéliales dérivées de la moelle osseuse (EC) ont été acquises auprès de Cell Biologics (numéro de catalogues RA-6221). Les cellules ont été cultivées dans un milieu EGM-2 MV (Lonza-Verviers, France) sur des plaques recouvertes de gélatine (2%) contenant tous les suppléments du kit et 5 % (v/v) de sérum de veau foetal (Gibco Life Technologies, Karlsruhe, Allemagne) et incubées à 37°C en atmosphère humide, avec 5 % de CO₂. Les cellules ont été transférées sur une autre plaque recouverte de gélatine (2 %) lorsqu'elles ont atteint 90% de confluence.

Les trois types cellulaires ont été étudiés sur les hydrogels de l'invention en raison de leur intérêt en ingénierie tissulaire : (i) les BMSC en raison de leur potentiel de différenciation ostéoblastique, (ii) les EC pour leur rôle majeur dans l'angiogenèse, et (iii) les neurones sensoriels pour démontrer la capacité du tissu nerveux à adhérer et à permettre la pousse des neurites dans les hydrogels.

Chaque type cellulaire a été caractérisé avant sa culture dans les hydrogels de l'invention.

### Activité métabolique cellulaire de chaque type cellulaire dans les hydrogels :

L'activité métabolique des cellules a été déterminée en utilisant un essai basé sur l'utilisation de la résazurine (O'Brien et al., Eur J Biochem 2000, 267, 5421). Brièvement. Les cellules ont été ensemencées dans des hydrogels à 7,5 % (p/v) à une densité de 20000 cellules/cm² dans une plaque 96 puits. 150 µL de milieu cellulaire contenant de la résazurine (0,01 mg/mL) ont été ajoutés à chaque puits et la microplaque a été incubée à 37°C pendant 3h. Puis, 100 µL de surnageant ont été transférés dans une autre microplaque 96 puits, et la fluorescence a été mesurée (exc = 530 nm, em = 590 nm, Victor X3, Perkin Elmer). L'activité métabolique a été mesurée aux jours 4 et 7 (n=5).

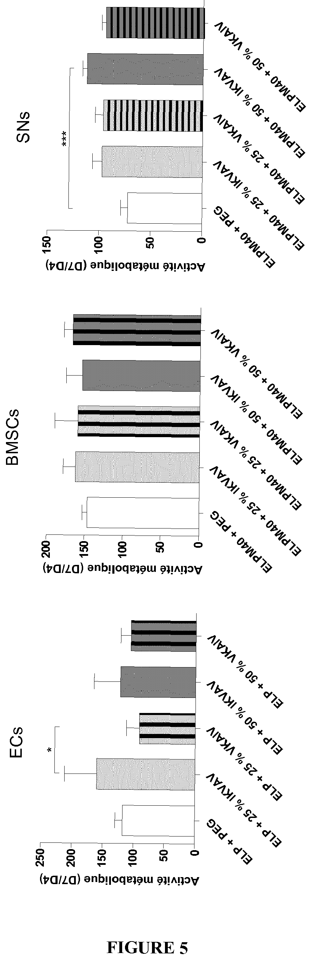

La figure 5 présente les résultats obtenus. Les hydrogels ont permis l'attachement et la culture des différentes cellules primaires testées sans effet cytotoxique. Pour les EC, l'activité métabolique était plus importante dans l'hydrogel ELPM40 + 25 % IKVAV, par rapport au contrôle de peptide aléatoire correspondant. Pour les SN, l'hydrogel ELPM40 + 50 % IKVAV induit une activité métabolique supérieure par rapport à la composition ELPM40 + PEG.

D'autres expériences ont également montré que des hydrogels comprenant une autre séquence ELP associée à du PEG, n'induisent pas de réponse inflammatoire lorsqu'ils sont implantés de manière sous-cutanée chez la souris.

### Microscopie confocale des hydrogels contenant des cellules :

Les cellules BMSC, EC et SN ont été ensemencées dans des hydrogels à une concentration de 20000 cellules/cm² et cultivées pendant 7 jours.

Après culture, les SN ont été fixés à 4°C pendant 30 minutes par 4 % (p/v) de formaldéhyde, perméabilisés avec du triton X-100 à 0,1 % (v/v) pendant 30 minutes à 4°C, bloqués avec de la BSA 1% (p/v) pendant 1h. Un anticorps primaire anti tubuline beta III a été utilisé pour la détection des cellules. Pour les BMSCs et les EC, les filaments d'actine ont été marqués au moyen de phaloidine conjuguée avec de l'Alexa Fluor 568. La morphologie des SN a été visualisée en utilisant un microscope confocal (SPE, Leica Microsystems). La quantification de la longueur des neurites a été réalisée en utilisant le logiciel ImageJ, avec l'outil « simple neurite tracer ». Les neurites ont été mesurés en traçant un trajet à partir du soma jusqu'à l'extrémité visible, puis longueur a été convertie en µm.

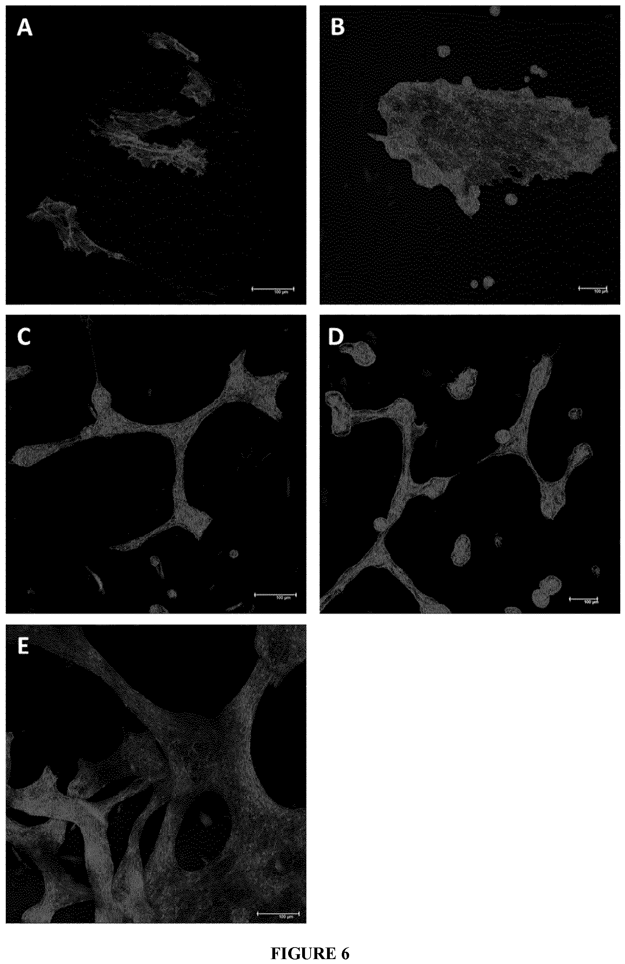

Après 7 jours de culture, les EC ont pénétré les hydrogels et y ont formé des structures ramifiées stables (figure 6). Ces structures sont importantes pour la délivrance d'oxygène et de nutriments au tissu vascularisé. Nous avons pu observer que les structures ramifiées se sont formées dans l'hydrogel ELPM40 + 50% IKVAV et dans les deux compositions comprenant le peptide aléatoire. Bien que la séquence aléatoire du peptide d'adhésion n'ait pas la même fonction d'adhésion que le peptide IKVAV, dans les compositions d'hydrogels contenant le peptide aléatoire nous n'avons pas observé de différence morphologique par rapport à la composition ELPM40 + 50% IKVAV. Il est possible que ce phénomène soit dû à l'augmentation des charges positives due aux résidus lysine.

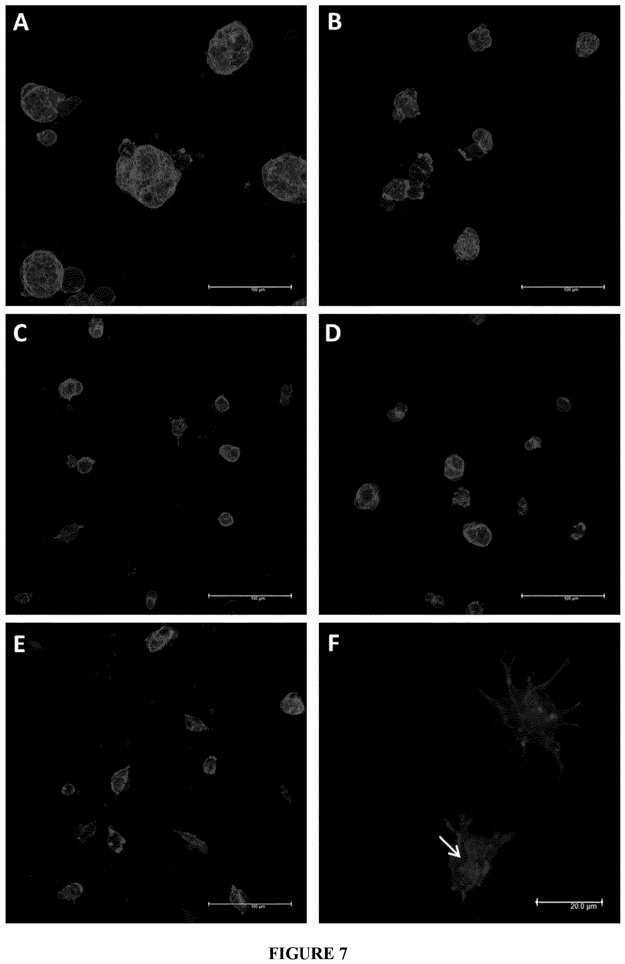

Lorsque les cellules BMSC ont été associées aux hydrogels, elles se sont développées en agrégats sphéroïdes pour toutes les compositions (figure 7). Notre étude montre que, pour la composition ELPM40 + 50 % IKVAV, certaines BMSC sont entrées dans la structure de l'hydrogel, ont formé des ramifications. Quelques cellules étaient binucléées, avec une connexion discrète entre les deux noyaux, suggérant que les cellules peuvent se diviser à l'intérieur du gel (figure 7F).

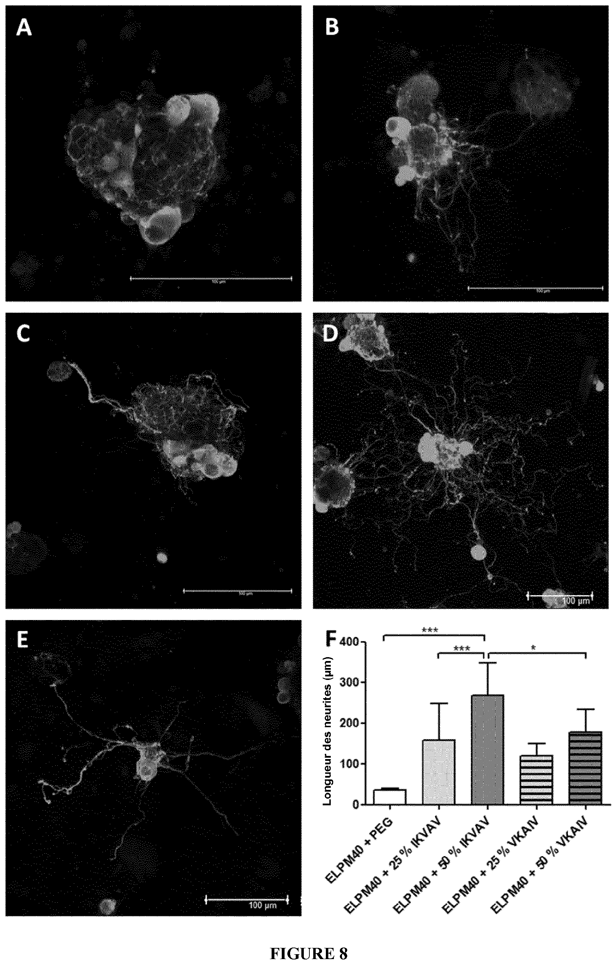

S'agissant des SN, les cellules ont présenté un comportement distinct selon la composition d'hydrogel (figure 8). Dans ELPM40 + PEG, les cellules ont formé des agrégats plus grands, et les neurites n'étaient pas dispersés dans la structure de l'hydrogel, mais entouraient plutôt le corps cellulaire (figure 8A). Lorsque la séquence IKVAV est ajoutée à la composition d'hydrogel, les neurites peuvent s'étendre. Dans la composition ELPM40 + 50 % IKVAV, les cellules ont adopté une distribution plus dispersée avec un réseau complexe de neurites, vaste et ramifié par comparaison avec les autres compositions (figure 8D). La mesure de la longueur des neurites confirme cette observation morphologique. En effet, les SN cultivées dans la composition ELPM40 + 50 % IKVAV ont pu former des neurites plus longues (figure 8F).

Il est important de noter que les milieux de culture utilisés dans cette étude ne contiennent pas de facteurs de croissance (notamment NGF), ceci dans le but d'analyser l'impact spécifique de l'hydrogel sur la structure et l'expansion des neurites. D'après nos résultats, la longueur moyenne des neurites dans la composition ELPM40 + 50% IKVAV est de 266,44 ± 63,95 µm, ce qui est équivalent à ce qui avait été mesuré dans d'autres études faites en 2D où le milieu de culture était supplémenté en NGF.

### Analyse moléculaire des marqueurs ostéo-spécifiques exprimés dans les BMSC dans l'hydrogel :

Les ARNs totaux ont été extraits des BMSC en utilisant le kit RNeasy^{®} Plus Micro Kit (Qiagen, Hilden, Allemagne) selon le protocole du fabricant. 100 ng d'ARNs totaux obtenu à partir de 4 puits ont été reverse transcrits en cDNA en utilisant le kit Maxima Reverse Transcriptase (Thermo Scientific^{™}, Thermo Fisher Scientific, Waltham, MA, USA), selon le protocole du fabricant. Des réactions de RT-PCR ont été réalisées, en utilisant le système CFX Connect^{™} Real-Time PCR Detection System (Bio-Rad Laboratories, Hercules, CA, USA) et analysées par le logiciel CFX Manager^{™}, version 3.0 (Bio-Rad Laboratories). Les amorces utilisées sont les suivantes (SEQ ID NO : 1 à 12) :
*Runx2* F: 5' CCTTCCCTCCGAGACCCTAA 3' et R: 5' ATGGCTGCTCCCTTCTGAAC 3',
*Sp7* F: 5' TGCTTGAGGAAGAAGCTCACTA 3' et R: 5' GGGGCTGAAAGGTCAGTGTA 3',
*Ctnnb1* (βcat) F: 5' GAAAATGCTTGGGTCGCCAG 3' et R: 5' CGCACTGCCATTTTAGCTCC 3',
*Bsp1* (Opn) F: 5' GAGTTTGGCAGCTCAGAGGA 3' et R: TCTGCTTCTGAGATGGGTCA 3',
*Smad1* F: 5' ATGGACACGAACATGACGAA 3' et R: 5' GCACCAGTGTTTTGGTTCCT 3',
*Rplp0* F: 5' CACTGGCTGAAAAGGTCAAGG 3' et 5' GTGTGAGGGGCTTAGTCGAA 3', et
*Tek* F: 5' CCACAGATAGAGGATTTGCCAG 3' et R: 5' AAGTCATTTGGTTGGAGCACTG 3'.

L'expression a été quantifiée en utilisant les valeurs de cycle seuil (Ct) et les niveaux d'expression des ARNm ont été calculés selon la méthode 2^{-DDCt}.

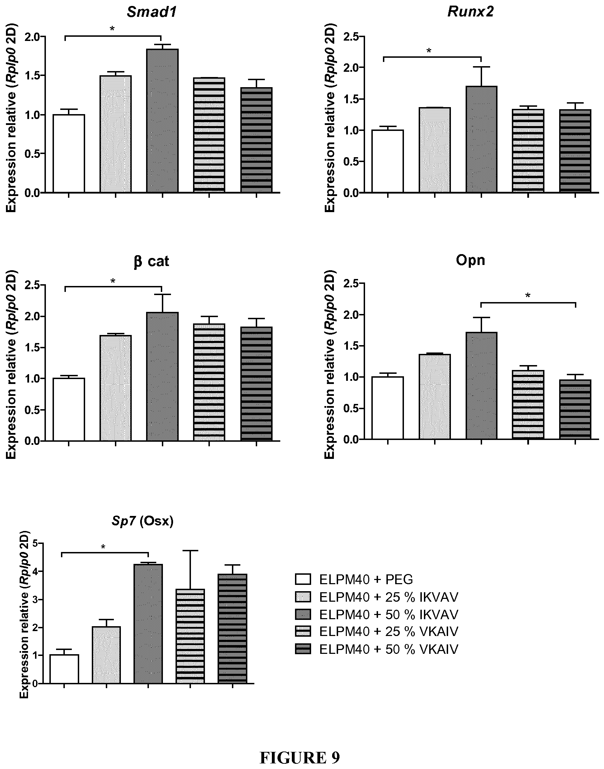

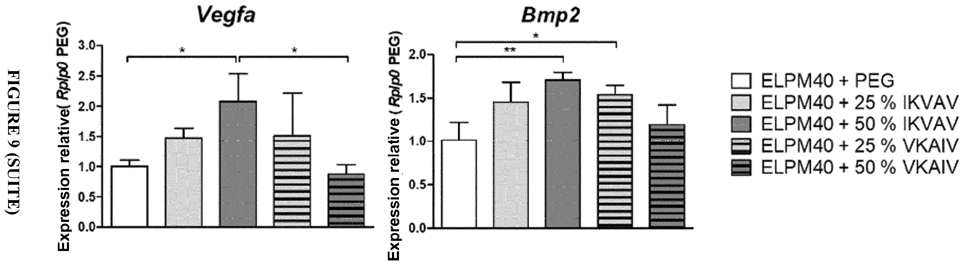

Pour déterminer si les compositions d'hydrogels peuvent supporter la différenciation ostéogénique des BMSC, nous avons analysé un groupe de marqueurs ostéogéniques de la différenciation précoce *(Runx2* et *Sp7*) et de la différenciation tardive (Opn), ainsi que des gènes liés au déclenchement des voies de signalisation ostéogéniques (*Smad1* et βcat). Par ailleurs, ont été analysés des facteurs clés pour le processus de réparation osseuse, qui induisent une vascularisation et la différenciation ostéoblastique: *vegfa* et *bmp2,* respectivement (Figure 9).

Après 7 jours de culture dans un milieu ostéogénique, nous observons une augmentation de l'expression de *Runx2* et *Sp7* dans la composition ELPM40 + 50 % IKVAV par rapport à ELPM40 + PEG, et l'expression d'Opn est augmentée dans la composition ELPM40 + 50 % IKVAV par rapport au contrôle de peptide aléatoire (figure 9). Lorsque nous avons analysé les voies de signalisation qui pourraient déclencher la différenciation ostéogénique, l'expression de *Smad1* et βcat a été augmentée dans ELPM40 + 50 % IKVAV par rapport à ELPM40 + PEG, suggérant que les deux voies de signalisation jouent un rôle dans la différenciation des BMSC associée à ces hydrogels. Ces résultats montrent donc que les peptides ELP peuvent représenter de très bons substrats favorisant l'ostéogenèse des BMSC. Par ailleurs, les niveaux d'expression de tous les gènes testés dans cette étude ont augmenté de manière dose-dépendante par rapport à la concentration du peptide IKVAV. La sur-expression des gènes qui déclenchent et agissent directement à différents stades de la différenciation ostéogénique supporte le rôle de la composition ELPM40 + 50 % IKVAV dans la différentiation des cellules BMSC vers le lignage ostéogénique. Le facteur angiogénique Vegfa a vu son expression augmentée avec la composition ELPM40 + 50 % IKVAV par rapport à ELPM40 + PEG ou ELPM40 + 50 % VKAIV. L'expression de Bmp2 a été augmentée en présence de ELPM40 + 25 %. De manière tout à fait intéressante, l'expression de tous les gènes étudiés à été augmentée avec la composition ELPM40 + 50 % IKVAV par rapport à la composition ELPM40 + PEG, et cette augmentation était proportionnelle à la concentration de IKVAV.

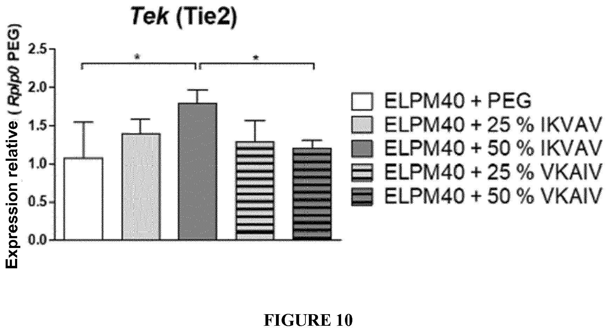

Afin de déterminer le potentiel pro-angiogénique des hydrogels de l'invention, des cellules endothéliales primaires de moëlle osseuse de rat ont été cultivées dans différentes compositions d'hydrogel. Après 7 jours de culture, l'expression de *Tek,* qui joue un rôle au cours de la formation des vaisseaux, a été évaluée (figure 10). Une sur-expression de *Tek* a été observée avec la composition comprenant 50 % de IKVAV par rapport à celles contenant du PEG ou le peptide aléatoire VKAIV (p<0,05).

Enfin, nous avons implanté de manière sous-cutanée la composition ELPM40 + 50 % IKVAV ou VKAIV (Figure 11). Aucune n'a déclenché de signaux majeurs de l'inflammation, comme l'atteste l'absence de cellules géantes multinuclées. Lorsque les vaisseaux sanguins ont été quantifiés dans la région entourant la zone d'implantation, nous avons observé une densité de vaisseaux supérieure avec les composition contenant IKVAV par rapport à celle contenant le peptide VKAIV après 26 jours d'implantation, la densité de vaisseaux augmentant avec le temps. Ces résultats sont supportés par les données obtenues *in vitro* et l'expression des gènes des cellules endothéliales, montrant une augmentation de l'expression de *Tek* dans les compositions contenant 50 % de IKVAV.

En conclusion, nous avons produit des hydrogels fonctionnels à base d' ELP-M(alcène)-40, SH-PEG et d'un peptide synthétique comprenant la séquence d'adhésion IKVAV. Ces hydrogels ont pour avantage d'avoir des propriétés rhéologiques finement adaptables. Pour les besoins de la présente étude, les hydrogels sélectionnés ont une concentration massique adaptée à la croissance des SN. Ces hydrogels sont par ailleurs dégradable *in vitro,* et ont une structure poreuse.

L'évaluation biologique a permis de montrer que les hydrogels de l'invention supportent la culture de cellules EC, BMSC et SN, que ces cellules ont pu migrer à l'intérieur de la structure d'hydrogel après 7 jours de culture, et que nous n'avons pas observé d'effet cytotoxique des hydrogels. S'agissant du potentiel de vascularisation, les EC ont pu former des structures ramifiées stables dans des compositions comprenant 50% de peptide d'adhésion. S'agissant du potentiel ostéogénique, la morphologie des BMSC était de type organisation sphéroïde dans toutes les compositions, et lorsque les cellules ont été cultivées dans une composition ELPM40 + 50% IKVAV, un ensemble de gènes importants pour la différenciation ostéogénique a été surexprimé. Enfin, s'agissant du potentiel de neurotisation, les SN cultivés dans la composition ELPM40 + 50% IKVAV présentaient un réseau de neurites plus complexe et une longueur de neurites plus importante, cette dernière étant comparable à celle obtenue dans d'autres cultures de neurites réalisées dans des milieux supplémenté en NGF, un facteur de croissance connu pour favoriser l'expansion des neurites.

Les données présentées dans cette demande montrent que la stratégie proposée, qui est basée sur des hydrogels spécifiques qui sont les premiers supports développés permettant une vascularisation, une ostéogenèse et une neurotisation sans présence d'autres facteurs cellulaires ou de facteurs de croissance, présente des caractéristiques intéressantes pour des applications biomédicales.

## Revendications

1. Hydrogel comprenant :
i) un polypeptide de type élastine comprenant au moins un résidu alcénylé avant formation de l'hydrogel; et
ii) un peptide capable de recruter des cellules neuronales et/ou endothéliales.

2. Hydrogel selon la revendication 1, comprenant en outre :
iii) un polymère de réticulation à terminaisons thiols avant formation de l'hydrogel.

3. Hydrogel selon la revendication 1 ou 2, dans lequel le peptide ii) est un peptide IKVAV, notamment un peptide de formule Cys-{Beta-Ala}-Ile-Lys-Val-Ala-Val-{Beta-Ala}-Cys

4. Hydrogel selon l'une quelconque des revendications 1 à 3, **caractérisé en ce que** le polypeptide de type élastine est un polypeptide comprenant au moins une occurrence de la séquence VPGMG.

5. Hydrogel selon l'une quelconque des revendications 1 à 4, **caractérisé en ce que** le polypeptide de type élastine est le polypeptide MGTELAAASEFTHMW[VPGMG]₂₀ (ELP20), le polypeptide MW[VPGVGVPGMG(VPGVG)₂]5 (ELPM20) ou le polypeptide MW[VPGVGVPGMG(VPGVG)₂]₁₀ (ELPM40).

6. Hydrogel selon l'une quelconque des revendications 2 à 5, **caractérisé en ce que** polymère de réticulation à terminaisons thiols est un polymère multibranches.

7. Hydrogel selon l'une quelconque des revendications 2 à 6, **caractérisé en ce que** le polymère de réticulation est un poly(éthylène glycol) à 4 branches à terminaisons thiols, ayant notamment une masse moléculaire moyenne comprise entre 10 et 30 kDa en masse, notamment une masse moléculaire moyenne de 20 kDa en masse.

8. Hydrogel selon l'une quelconque des revendications 2 à 7, **caractérisé en ce que** le polymère de réticulation à terminaisons thiols, le polypeptide type élastine comprenant un résidu méthionine alkénylé, et le peptide IKVAV sont présents en un ratio thiol / alkène équimolaire.

9. Hydrogel selon l'une quelconque des revendications 1 à 8, **caractérisé en ce que** la concentration de l'hydrogel est comprise entre 5 et 15% en masse volumique (p/v), en particulier entre 7 et 8% (p/v).

10. Hydrogel selon l'une quelconque des revendications 1 à 9, **caractérisé en ce que** le module de conservation G' de l'hydrogel est compris entre 1 et 1,5 kPa.

11. Hydrogel selon l'une quelconque des revendications 1 à 9, comprenant en outre au moins un agent actif sur le plan biologique, notamment au moins un facteur de croissance.

12. Support tridimensionnel capable d'héberger des cellules d'intérêt, **caractérisé en ce qu'**il comprend un hydrogel selon l'une quelconque des revendications 1 à 11.

13. Hydrogel selon l'une quelconque des revendications 1 à 11 ou support selon la revendication 12, pour son utilisation en tant que médicament.

14. Hydrogel selon l'une quelconque des revendications 1 à 11 ou support selon la revendication 12, pour son utilisation dans une méthode de régénération osseuse.

15. Méthode *in vitro* de culture cellulaire, comprenant la mise en culture de cellules dans un hydrogel tel que défini dans l'une quelconque des revendications 1 à 11 ou dans un support selon la revendication 12.

## Patentansprüche

1. Hydrogel umfassend:
i) ein Polypeptid vom Elastin-Typ, das vor der Bildung des Hydrogels mindestens einen alkenylierten Rest umfasst;
und
ii) ein Peptid, das in der Lage ist, neuronale und/oder Endothelzellen zu rekrutieren.

2. Hydrogel nach Anspruch 1, weiterhin umfassend:
iii) ein vernetzendes Polymer mit Thiol-Endungen vor der Bildung des Hydrogels.

3. Hydrogel nach Anspruch 1 oder 2, bei dem das Peptid ii) ein IKVAV-Peptid ist, insbesondere ein Peptid der Formel Cys-{Beta-Ala}-Ile-Lys-Val-Ala-Val-{Beta-Ala}- Cys.

4. Hydrogel nach einem der Ansprüche 1 bis 3, **dadurch gekennzeichnet, dass** das Polypeptid vom Elastin-Typ ein Polypeptid ist, das mindestens ein Vorkommen der VPGMG-Sequenz umfasst.

5. Hydrogel nach einem der Ansprüche 1 bis 4, **dadurch gekennzeichnet, dass** das Polypeptid vom Elastin-Typ das Polypeptid MGTELAAASEFTHMW[VPGMG]₂₀ (ELP20), das Polypeptid MW[VPGVGVPGMG(VPGVG)₂]5 (ELPM20) oder das Polypeptid MW[VPGVGVPGMG(VPGVG)₂]10 (ELPM40) ist.

6. Hydrogel nach einem der Ansprüche 2 bis 5, **dadurch gekennzeichnet, dass** das vernetzende Polymer mit Thiol-Endungen ein mehrfach verzweigtes Polymer ist.

7. Hydrogel nach einem der Ansprüche 2 bis 6, **dadurch gekennzeichnet, dass** das vernetzende Polymer ein Poly-(Ethylenglykol) mit 4 Verzweigungen mit Thiol-Endungen ist, insbesondere mit einer durchschnittlichen Molekularmasse zwischen 10 und 30 kDa an Masse, insbesondere eine durchschnittliche Molekülarmasse von 20 kDa Masse.

8. Hydrogel nach einem der Ansprüche 2 bis 7, **dadurch gekennzeichnet, dass** das vernetzende Polymer Thiol-Endungen hat, das Polypeptid vom Elastin-Typ einen alkenylierten Methioninrest umfasst und das IKVAV-Peptid in einem äquimolaren Thiol/Alken-Verhältnis vorliegen.

9. Hydrogel nach einem der Ansprüche 1 bis 8, **dadurch gekennzeichnet, dass** die Konzentration des Hydrogels zwischen 5 und 15 % der Dichte (w/v), insbesondere zwischen 7 und 8 % (w/v) beträgt.

10. Hydrogel nach einem der Ansprüche 1 bis 9, **dadurch gekennzeichnet, dass** der Erhaltungsmodul G' des Hydrogels zwischen 1 und 1,5 kPa liegt.

11. Hydrogel nach einem der Ansprüche 1 bis 9, weiterhin umfassend mindestens einen biologisch aktiven Wirkstoff, insbesondere mindestens einen Wachstumsfaktor.

12. Dreidimensionaler Träger, der in der Lage ist, interessierende Zellen aufzunehmen, **dadurch gekennzeichnet, dass** er ein Hydrogel nach einem der Ansprüche 1 bis 11 umfasst.

13. Hydrogel nach einem der Ansprüche 1 bis 11 oder Träger nach Anspruch 12, zur Verwendung als Medikament.

14. Hydrogel nach einem der Ansprüche 1 bis 11 oder Träger nach Anspruch 12, zur Verwendung in einem Verfahren zur Knochenregeneration.

15. In-vitro-Zellkulturverfahren, umfassend das Kultivieren von Zellen in einem Hydrogel wie definiert in einem der Ansprüche 1 bis 11 oder in einem Träger gemäß Anspruch 12.

## Claims

1. A hydrogel comprising:
i) an elastin-like polypeptide comprising at least one alkenylated residue before formation of the hydrogel; and
ii) a peptide capable of recruiting neuronal and/or endothelial cells.

2. The hydrogel according to Claim 1, also comprising:
iii) a crosslinking polymer with thiol end groups before formation of the hydrogel.

3. The hydrogel according to Claim 1 or 2, wherein the peptide ii) is an IKVAV peptide, in particular a peptide of formula Cys-{Beta-Ala}-Ile-Lys-Val-Ala-Val-{Beta-Ala}-Cys.

4. The hydrogel according to any one of Claims 1 to 3, **characterized in that** the elastin-like polypeptide is a polypeptide comprising at least one occurrence of the sequence VPGMG.

5. The hydrogel according to any one of Claims 1 to 4, **characterized in that** the elastin-like polypeptide is the MGTELAAASEFTHMW[VPGMG]₂₀ (ELP20) polypeptide, the MW[VPGVGVPGMG(VPGVG)₂]₅ (ELPM20) polypeptide or the MW[VPGVGVPGMG(VPGVG)₂]₁₀ (ELPM40) polypeptide.

6. The hydrogel according to any one of Claims 2 to 5, **characterized in that** the crosslinking polymer with thiol end groups is a multi-arm polymer.

7. The hydrogel according to any one of Claims 2 to 6, **characterized in that** the crosslinking polymer is a 4-arm poly(ethylene glycol) with thiol end groups, having in particular an average molecular weight between 10 and 30 kDa in weight, in particular an average molecular weight of 20 kDa in weight.

8. The hydrogel according to any one of Claims 2 to 7, **characterized in that** the crosslinking polymer with thiol end groups, the elastin-like polypeptide comprising an alkenylated methionine residue, and the IKVAV peptide are present in an equimolar thiol/alkene ratio.

9. The hydrogel according to any one of Claims 1 to 8, **characterized in that** the concentration of the hydrogel is between 5 and 15% by density (w/v), in particular between 7 and 8% (w/v).

10. The hydrogel according to any one of Claims 1 to 9, **characterized in that** the storage modulus G' of the hydrogel is between 1 and 1.5 kPa.

11. The hydrogel according to any one of Claims 1 to 9, also comprising at least one biologically active agent, in particular at least one growth factor.

12. A three-dimensional support capable of housing cells of interest, **characterized in that** it comprises a hydrogel according to any one of Claims 1 to 11.

13. The hydrogel according to any one of Claims 1 to 11 or the support according to Claim 12, for use as a medicament.

14. The hydrogel according to any one of Claims 1 to 11 or the support according to Claim 12, for use in a bone regeneration method.

15. An *in vitro* cell culture method, comprising the culturing of cells in a hydrogel as defined in any one of Claims 1 to 11 or in a support according to Claim 12.
